# EUROPEAN PATENT SPECIFICATION

(11) **EP 1 792 631 B1**
(45) Date of publication and mention of the grant of the patent: **15.04.2009**
(21) Application number: 05026070.2
(22) Date of filing: 30.11.2005
(51) Int. Cl.: A61L 2/20

(54) **Process for DNA decontamination**
Verfahren zur DNA-Dekontamination
Procédé pour la décontamination de DNA

(43) Date of publication of application: 06.06.2007
(73) Proprietor: Roche Diagnostics GmbH, 68305 Mannheim (DE); F.HOFFMANN-LA ROCHE AG, 4070 Basel (CH)
(72) Inventor: Engel, Wolf-Dieter, 82340 Feldafing (DE); Rainer, Alois, 81241 München (DE); Siedel, Joachim, 82347 Bernried (DE); Tgetgel, Armin, 82386 Huglfing (DE)

(56) References cited:
- WO-A-86/02842
- WO-A-20/05007884
- US-A- 3 068 064
- US-A- 4 770 851
- US-A- 5 472 702
- US-A- 5 749 203
- US-A1- 2002 119 074
- SEYMOUR S. BLOCK: "Sterilization, disinfection, and preservation" 2001, LIPPINCOTT , U.S.

## Description

### Field of Invention

The present invention provides a method to produce disposables for highly sensitive PCR analytics that are free from amplifiable nucleic acid contamination.

### Background of the Invention

Document US 5 749 203 discloses a conventional EtO sterilisation method and its typical process parameters concerning temperature, time, pressure and concentration. In particular, US 5 749 203 comprises a method to alkylate DNA molecules at surfaces of disposables comprising providing said disposable in a temperature controlled pressure chamber, adjusting the parameters of said temperature controlled pressure chamber, gassing said disposable with ethylene oxide, rinsing said disposable with cleaning gas and desorbing of ethylene oxide. Further, the pressure chamber is adjusted to 40 - 500 mbar and more than 15 °C; gassing of disposables is performed with a concentration of at least 100 g/m3, whereby gassing of disposables is performed for at least 1 hour, desorbing of ethylene oxide is performed at atmospheric pressure and the disposables are surrounded by packing material and are products made of glass, plastic, ceramics or metal together with the respective packing materials of said products.

WO 2005/007884 teaches that EtO may be employed for complete DNA inactivation prior to the use of an item in a PCR process in order to avoid falsified results by DNA contamination.

Biological contamination is a common problem in many fields of science, health care and industry. Especially for the food industry and for medical services the absence of potentially pathogenic germs like bacteria, virus and fungi on devices has to be ascertained, even though an environment completely free of germs is not achievable. With respect to analytical science such biological contaminations may have drastic effects on measurements leading to false results.

There are many techniques known to someone skilled in the art that are suitable for the sterilization of devices, whereas the proper method depends on the material of the device and the required extent of sterility. Known sterilization methods are e.g. electromagnetic radiation, steam, hot air, hydrogen peroxide plasma or treatment with certain other chemicals.

All sterilization methods have in common that the germs are altered in such a way that reproduction is no longer possible. However, for devices and disposables that are used for analysis of nucleic acids, in particular for PCR applications, the mere sterility is not enough, because even dead germs may still have amplifiable DNA or RNA molecules.

A well known technique for the sterilization of thermal unstable materials comprises the use of ethylene oxide, because this substance is effective even at temperatures below 10 °C. Because ethylene oxide is gaseous at room temperature and atmospheric pressure, the substance can reach even devices that are surrounded by certain packing materials.

Ethylene oxide is known to act with biological material through alkylation producing irreversible damage to all components of the germs like nucleic acids, proteins and enzymes and therefore, further reproduction is prevented (sterilization).

Nevertheless, it has not been demonstrated so far that ethylene oxide treatment does not only lead to a sterilization, but simultaneously also enables for the complete inactivation (= decontamination) of nucleic acid (DNA, RNA), even if said nucleic acid is still contained within e.g. microbial germs.

Within the present invention it was found that ethylene oxide treatment of disposables can be performed in such an effective way that no amplifiable nucleic acid contamination remains and that said disposables are therefore applicable even for highly sensitive PCR analytics.

### Description of the Invention

Many alternatives to avoid the amplification of nucleic acid contaminations are known to someone skilled in the art, but none of these alternatives are applicable to eliminate false nucleic acid amplification results originating from nucleic acid contaminations on surfaces of e.g. thermolabile disposables.

Therefore, the present invention is directed to a method according to claim 1.

Throughout the present invention the alkylation of DNA means the chemical modification of DNA molecules by adding alkyl groups. Such an alkylation is known also for other cellular constituents of organisms, like proteins and enzymes. As a result of the alkylation of cellular constituents, cell reproduction is prevented and cell death ensues.

It is known that gaseous ethylene oxide (C₂H₄O) is a powerful alkylating agent for cellular constituents of organisms, if the correct conditions are provided. These include an adequate concentration of ethylene oxide and a necessary level of water within the organism. Since the process is a chemical reaction it is temperature dependent, whereas the reaction rate is increasing with temperature. These properties define the key characteristics of an ethylene oxide sterilization process.

The parameters of the ethylene oxide treatment can be controlled by using a temperature controlled pressure chamber. Such a pressure chamber is preferably made out of stainless steel equipped with a vacuum pump to control the vacuum, a chamber jacket to circulate hot liquid or air in order to adjust the temperature and a device to inject steam to the chamber affecting the humidity within the chamber.

After the gassing of the disposables in step c), ethylene oxide is adsorbed at the surface of the disposables as well as of the chamber itself. Due to the toxicity of ethylene oxide, it is necessary to remove the chemical prior to the removal of the disposables from the temperature controlled pressure chamber. This is achieved by using a cleaning gas replacing the ethylene oxide atmosphere within the pressure chamber. Each replacement of the atmosphere within the pressure chamber by fresh cleaning gas is called a rinsing step within the present invention.

Note that mere sterility of devices and disposables may be insufficient for PCR applications, because even dead germs may still have amplifiable DNA or RNA molecules causing false amplification results. Therefore, the parameters of the ethylene oxide treatment such as time, temperature, pressure, humidity and ethylene oxide concentration have to be adapted towards the sufficient suppression of DNA amplification.

Prior to the gassing step air is removed from the pressure chamber using a vacuum pump to an initial pressure of about 40 - 500 mbar. Afterwards, ethylene oxide is injected to the chamber and consequently, the pressure within the chamber increases.

The temperature is an important parameter of the ethylene oxide treatment, because it is known that the rate of alkylation is strongly depending on the reaction temperature. Although ethylene oxide develops its alkylating power already at around 15 °C, the necessary incubation time can be reduced to the sixth part, if the temperature is increased from 20 to 60 °C. Note that ethylene oxide starts to fluidify at temperatures between 10 and 15 °C.

According to the present invention, said gassing of disposables is performed with a concentration of at least 100 g/cm³, preferably between 300 - 1200 g/cm³ at 400 - 800 mbar.

With respect to the concentration of ethylene oxide two aspects have to be considered. On the one hand, the efficiency of DNA alkylation increases with increasing ethylene oxide concentration and therefore, high ethylene oxide concentrations are preferred. Note that this is only true up to a certain concentration, where a further increase in concentration no longer yields any improvement. On the other hand, ethylene oxide is toxic and therefore, increasing its amount adds higher health risks to the process.

In yet another preferred method according to the present invention, said gassing of disposables is performed for at least 1 hour, preferably between 3 - 8 hours.

The incubation time with ethylene oxide is strongly depending on the concentration. For example, the incubation time for a standard sterilization is reduced from 460 to 280 min, if the concentration is increased from 200 to 600 mg/cm³.

Humidity is another important parameter of the ethylene oxide treatment, because it is known that ethylene oxide needs water to develop its alkylating power. Especially with respect to bacterial spores having a dry cellular wall, the effect of ethylene oxide is inhibited, if no humidity is added to the process. The humidity within the chamber is adjusted by inserting a controlled amount of steam.

In a more preferred method according to the present invention, said humidity is adjusted incremental.

In this preferred embodiment of the present invention, the final humidity within the pressure chamber is adjusted incremental performing several humidification steps. After each humidification step of inserting a controlled amount of steam, the atmosphere within the chamber may adjust during a waiting period of several minutes. This procedure helps to avoid droplet formation and supports a homogeneous humidity. It is preferred to adjust the humidity by more than 3 incremental steps.

In still another preferred method according to the present invention, said humidification step is performed for 5 - 35 minutes at 80 - 600 mbar.

After reaching the final humidity, a homogeneous moisturization of the disposables is reached during an incubation time of between 5 - 35 minutes. During the humidification step, the pressure within the chamber is increased from the initial pressure to about 80 - 600 mbar due to the inserted steam.

In a preferred embodiment of the method according to the present invention, said cleaning gas is air.

As mentioned before, the ethylene oxide has to be removed from the disposables and the temperature controlled pressure chamber prior to the removal of the disposables. This is preferably performed by replacing the ethylene oxide atmosphere within the pressure chamber with air. The ethylene oxide itself is preferably converted to carbon dioxide via a catalytic conversion step.

In another preferred embodiment of the method according to the present invention, said rinsing in step d) is performed twice, preferably more than 3 times.

After the one or more rinsing steps, the disposables are finally stored at atmospheric pressure in order to desorb remaining ethylene oxide to an unperilous level.

In yet another preferred embodiment of the method according to the present invention, said desorbing of ethylene oxide in step e) is performed at atmospheric pressure, preferably for at least 5 hours.

Note that the desorbing of ethylene oxide is strongly depending on the porosity and on the chemical composition of the disposable. In addition, it is known that ethylene oxide may form stable bonds with certain plastic materials and can not be adsorbed in this case at all. Therefore, it is possible to detect the presence of ethylene oxide at gassed disposables even after several weeks, but the amount of desorbing ethylene oxide is far below a harmful dose and has no influence on the subsequent PCR performance.

The following examples and figures are provided to aid the understanding of the present invention, the true scope of which is set forth in the appended claims. It is understood that modifications can be made in the procedures set forth without departing from the spirit of the invention.

### Description of the Figures

- **Figure 1:**: PCR plot illustrating the alkylation efficiency of an ethylene oxide treatment of plastic disposables contaminated with spores.
- **Figure 2:**: PCR plot illustrating the alkylation efficiency of an ethylene oxide treatment of glass disposables contaminated with spores.
- **Figure 3:**: PCR plots comprising amplification curves performed with (a) and without ethylene oxide treatment (b) of disposables using a SybrGreen assay.
- **Figure 4:**: PCR plots comprising amplification curves performed with (a) and without ethylene oxide treatment (b) of disposables using a hybridization probe assay.

### Example 1:

### Gassing with ethylene oxide

The disposables were arranged in a 33 m³ stainless steel sterilization chamber and the chamber was set to initially 50 mbar and 48 °C. Afterwards, the humidity of the chamber was incrementally (4 injections of steam) adjusted to 65 %, whereas the pressure rose to 90 mbar. After a humidification time of 35 minutes, the chamber was filed with ethylene oxide (throughout the experiments gas with a purity of 98 % was used) to a final concentration of 800 g/m³, whereas the pressure inside of the chamber rose to 500 mbar. After an incubation time with ethylene oxide of 4 hours, the chamber was rinsed 4 times with air. Finally, the disposables were stored for 7 days under constant ventilation in order to desorb the remaining ethylene oxide.

### Example 2:

### Decontamination efficiency at plastic disposables

To test the efficiency of the ethylene oxide treatment towards alkylation of nucleic acids for the purpose of avoiding amplification, a contamination was simulated by placing 10⁶ bacterial spores (*Bacillus subtillis*) on plastic disposables (polypropylene). Afterwards said plastic disposables were wrapped in plastic foil and treated with ethylene oxide as explained in example 1.

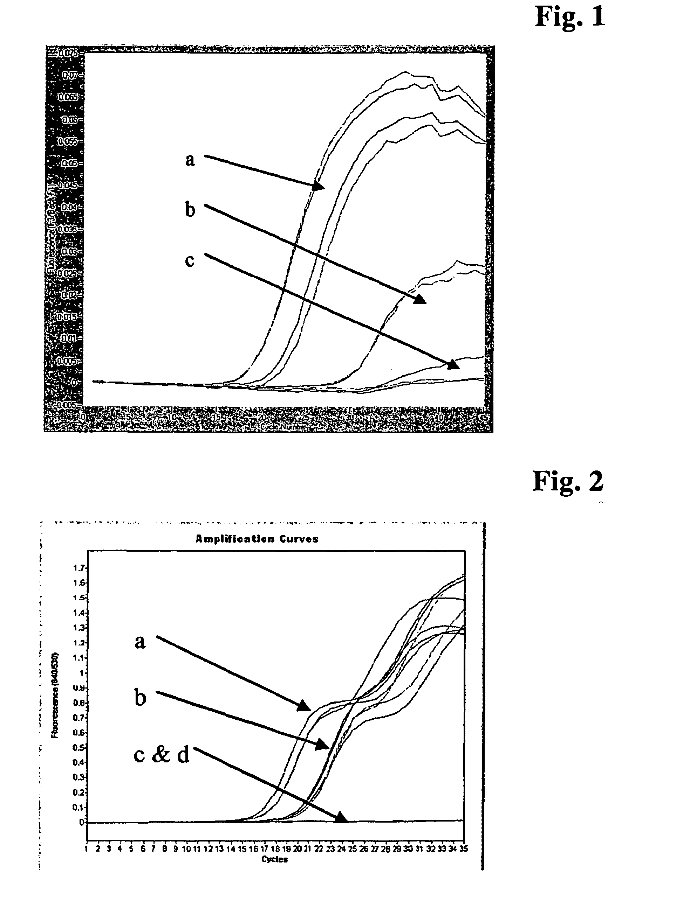

Then the plastic foil was removed and the ethylene oxide treated germs were eluted in 500 µl PCR-grade water. The eluat was then added directly into LightCycler^{®} glass capillaries and a PCR amplification was performed using a LightCycler^{®} 2.0 apparatus and the amplification kit LightCycler^{®} FastStart DNA Master HybProbe (all of Roche Diagnostics GmbH; the quality of the kit reagents were improved in order to further remove traces of intrinsic DNA contamination). Note that the lysis and the DNA release from the bacterial spores occurs automatically during the hot start phase of the subsequent PCR. The primers of the assay were designed to amplify a highly conserved region of the bacterial gene sequence coding for the 16s rRNA as known from the literature. To calculate the decontamination efficiency, an analogous sample without the ethylene oxide treatment was prepared and amplified as well. The amplification curves of this experiment are plotted in Figure 1 (a: without ethylene oxide treatment, b: with ethylene oxide treatment, c: no-template-control) and a depletion factor of about 5000 was gained.

### Example 3:

### Decontamination efficiency at glass disposables

To test the efficiency of the ethylene oxide treatment towards alkylation of nucleic acids for the purpose of avoiding amplification, a contamination was simulated by placing 2x10⁶ bacterial spores (*Bacillus subtillis*) on glass disposables (100 µl capillaries for the LightCycler^{®} 2.0, Roche Diagnostics GmbH). Afterwards said glass disposables were wrapped in plastic foil and treated with ethylene oxide as explained in example 1.

Then the plastic foil was removed and the ethylene oxide treated spores were eluted by adding PCR-grade water to the capillaries. Afterwards, a PCR amplification was performed in the same capillaries using a LightCycler^{®} 2.0 apparatus and the amplification kit LightCycler^{®} FastStart DNA Master HybProbe (both of Roche Diagnostics GmbH) analogous to Example 2. To verify the decontamination efficiency, additionally a sample without the ethylene oxide treatment as well as an ethylene oxide treated sample with subsequent addition of bacterial plasmids were prepared and amplified.

The amplification curves of this experiment are plotted in Figure 2 (a: disposables without ethylene oxide treatment, b: disposables with ethylene oxide treatment and subsequent addition of 10⁴ copies of a *Bacillus subtillis* plasmid as reaction control, c: disposables with ethylene oxide treatment, d: no-template-control) and a decontamination efficiency of close to 100 % was reached.

### Example 4:

### Impact of ethylene oxide on the PCR performance

In order to verify the influence of remaining ethylene oxide on the performance two different PCR assay were performed.

First, the influence of ethylene oxide was tested for a SybrGreen real-time PCR amplification of a dilution series (10⁵, 10⁴, 10³, 10² and 10 copies) of the human house-keeping gene G6PDH using the LightCycler^{®} FastStart DNA Master SYBR Green I according to the specifications of the manufacturer (Roche Diagnostics GmbH) and a primer pair specific for the G6PDH gene. For this experiment the primers from the LightCycler^{®}-t(9;22) Quantification Kit (Roche Diagnostics GmbH) were applied and a standard of the same kit was used to produce the dilution series of the house-keeping gene.

Second, the influence of ethylene oxide was tested for a real-time PCR amplification of a dilution series of a microbial plasmid (10⁶, 10⁵, 10⁴, 10³, 10² 10 and 1 copies) using hybridization probes. For this experiment the LightCycler^{®}-Parvovirus Quantification Kit (Roche Diagnostics GmbH) was used according to the specifications of the manufacturer, whereas for the dilution series of the microbial plasmid the standard of the kit was used.

For both experiments, untreated LightCycler^{®} 480 multiwell plates with 384 wells (Roche Diagnostics GmbH) were compared with multiwell plates treated with ethylene oxide (see Example 1) with respect to the respective PCR amplifications.

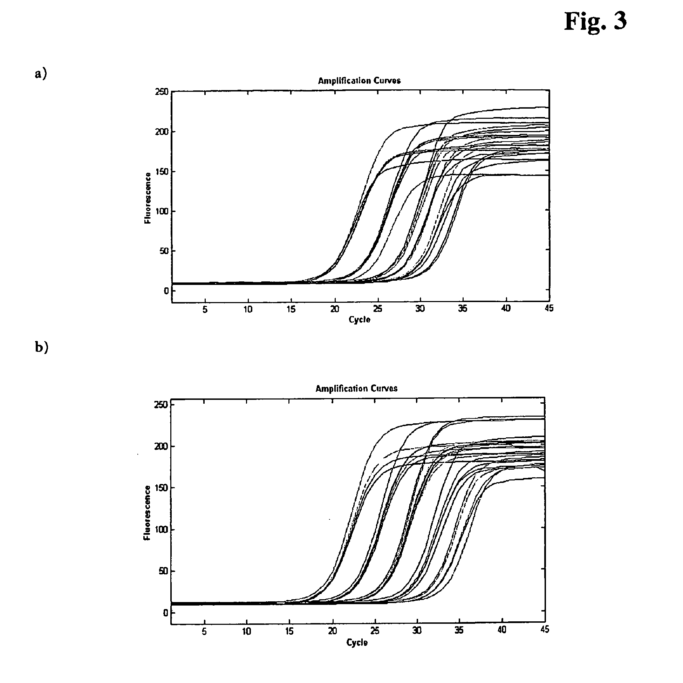

The amplification curves of the first experiment are plotted in Figure 3 (a: treated with ethylene oxide, b: untreated). The following table comprises the cp-values of the experiment, indicating that no difference was detected in the subsequent PCR amplification.

| copies | Treated | untreated |
|---|---|---|
| 10⁵ | 19,54 | 19,03 |
| 10⁴ | 23,06 | 22,52 |
| 10³ | 26,77 | 25,89 |
| 10² | 28,68 | 25,97 |
| 10 | 30,19 | 29,16 |

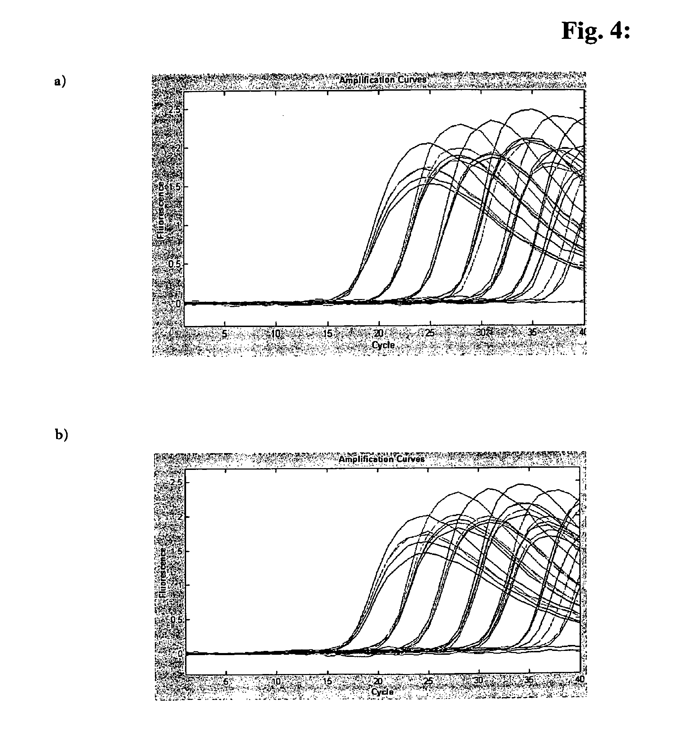

The amplification curves of the second experiment are plotted in Figure 4 (a: treated with ethylene oxide, b: untreated). The following table comprises the cp-values of the experiment, indicating that no difference was detected in the subsequent PCR amplification.

| copies | Treated | untreated |
|---|---|---|
| 10⁶ | 16,53 | 16,56 |
| 10⁵ | 20,17 | 20,16 |
| 10⁴ | 23,56 | 23,60 |
| 10³ | 27,15 | 27,01 |
| 10² | 30,18 | 30,02 |
| 10 | 33,53 | 33,76 |
| 1 | --- | --- |

## Claims

1. A method to produce disposables for highly sensitive PCR analytics that are free from amplifiable nucleic acid contaminations by alkylating DNA molecules at surfaces of said disposables, said method comprising
a) providing said disposable in a temperature controlled pressure chamber,
b) adjusting the temperature to more than 15 °C and the pressure of said temperature controlled pressure chamber to 40 - 500 mbar,
c) adjusting the humidity 50 - 90 %,
d) gassing said disposable with ethylene oxide at a concentration of at least 100 g/m³,
e) rinsing said disposable with cleaning gas and
f) desorbing of ethylene oxide.

2. The method according to claim 1, whereas said temperature controlled pressure chamber is adjusted to between 45 - 55 °C.

3. The method according to claim 1 or 2, whereas said gassing of disposables is performed with a ethylene oxide concentration of between 300 - 1200 g/m³ at 400 - 800 mbar.

4. The method according to claims 1 - 3, whereas said gassing of disposables is performed for at least 1 hour, preferably between 3 - 8 hours.

5. The method according to claims 1-4, whereas said humidification in step c) is performed for 5 - 35 minutes at 80 - 600 mbar.

6. The method according to claims 1 - 5, whereas said desorbing of ethylene oxide in step f) is performed at atmospheric pressure, preferably for at least 5 hours.

## Patentansprüche

1. Methode zur Herstellung von Einwegartikeln für die hochempfindliche PCR-Analytik, die frei von amplifizierbaren Nukleinsäurekontaminationen sind, durch Alkylieren von DNA-Molekülen an Oberflächen der Einwegartikel, wobei die Methode Folgendes umfasst:
a) Bereitstellen des Einwegartikels in einer Druckkammer mit Temperaturkontrolle;
b) Einstellen einer Temperatur auf mehr als 15 °C und eines Drucks auf 40-500 mbar in der Druckkammer mit Temperaturkontrolle;
c) Einstellen der Luftfeuchtigkeit auf 50-90%;
d) Begasen des Einwegartikels mit Ethylenoxid bei einer Konzentration von mindestens 100 g/m³;
e) Spülen des Einwegartikels mit Reinigungsgas und
f) Desorbieren des Ethylenoxids.

2. Verfahren nach Anspruch 1, wobei die Druckkammer mit Temperaturkontrolle auf einen Wert zwischen 45 - 55 °C eingestellt wird.

3. Verfahren nach Anspruch 1 oder 2, wobei das Begasen der Einwegartikel mit einer Ethylenoxidkonzentration zwischen 300 - 1200 g/m³ bei 400 - 800 mbar erfolgt.

4. Verfahren nach Ansprüchen 1 - 3, wobei das Begasen der Einwegartikel über einen Zeitraum von mindestens 1 Stunde, vorzugsweise zwischen 3 - 8 Stunden, erfolgt.

5. Verfahren nach Ansprüchen 1 - 4, wobei die Luftbefeuchtung in Schritt c) für 5 - 35 Minuten bei 80 - 600 mbar erfolgt.

6. Verfahren nach Ansprüchen 1 - 5, wobei das Desorbieren von Ethylenoxid in Schritt f) bei Normaldruck, vorzugsweise über einen Zeitraum von mindestens 5 Stunden, erfolgt.

## Revendications

1. Procédé pour produire des articles à usage unique pour les procédés analytiques par PCR hautement sensibles qui sont exempts de contaminations de type acide nucléique amplifiable par alkylation de molécules d'ADN aux surfaces desdits articles à usage unique, ledit procédé comprenant
a) l'introduction dudit article à usage unique dans une chambre de pression à température contrôlée,
b) l'ajustement de la température au-dessus de 15°C et de la pression de ladite chambre de pression à température contrôlée à 40-500 mbars,
c) l'ajustement de l'humidité à 50-90%,
d) le gazage dudit article à usage unique avec de l'oxyde d'éthylène à une concentration d'au moins 100 g/m³,
e) le rinçage dudit article à usage unique avec du gaz de nettoyage et
f) la désorption de l'oxyde d'éthylène.

2. Procédé selon la revendication 1, dans lequel ladite chambre de pression à température contrôlée est ajustée entre 45-55°C.

3. Procédé selon la revendication 1 ou 2, dans lequel ledit gazage des articles à usage unique est réalisé avec une concentration en oxyde d'éthylène entre 300-1200 g/m³ à 400-800 mbars.

4. Procédé selon les revendications 1-3, dans lequel ledit gazage des articles à usage unique est réalisé pendant au moins 1 heure, de préférence entre 3-8 heures.

5. Procédé selon les revendications 1-4, dans lequel ladite humidification dans l'étape c) est réalisée pendant 5-35 minutes à 80-600 mbars.

6. Procédé selon les revendications 1-5, dans lequel ladite désorption de l'oxyde d'éthylène dans l'étape f) est réalisée à la pression atmosphérique, de préférence pendant au moins 5 heures.
